# EUROPEAN PATENT APPLICATION

(11) **EP 3 146 966 A1**
(43) Date of publication of application: **29.03.2017**
(21) Application number: 16197220.3
(22) Date of filing: 08.09.2014
(51) Int. Cl.: A61K 31/427, A61P 37/00, A61P 37/08, A61P 17/00, A61P 25/00

(54) **PIDOTIMOD FOR USE IN THE TREATMENT OF INFLAMMATION-ASSOCIATED DISEASES**

(30) Priority: 10.09.2013 WO PCT/EP2013/068701
(62) Divisional of application: 14761638.7
(71) Applicant: POLICHEM S.A., 1526 Luxembourg (LU)
(72) Inventor: CARUSO, Arnaldo, I-25123 Brescia (IT); FIORENTINI, Simona, I-25069 Villa Carcina (BS) (IT)
(74) Representative: Pistolesi, Roberto

(57) **Abstract**

The present invention relates to a novel use of the active ingredient Pidotimod, or its stereoisomers and/or physiologically acceptable salts thereof, to treat inflammation-associated diseases characterized by an aberrant hyperactivation of the non canonical NFkB pathway, such as allergic diseases, autoimmune diseases or other inflammatory diseases based on mechanisms different from allergic or autoimmune.

## Description

The present invention relates to a novel use of the active ingredient Pidotimod, or its stereoisomers and/or physiologically acceptable salts thereof, to treat inflammation-associated diseases characterized by an aberrant hyperactivation of the non canonical NFkB pathway, such as allergic diseases, autoimmune diseases or other inflammatory diseases based on mechanisms different from allergic or autoimmune.

### BACKGROUND

The recognition of microbial components during host infection is a key step in activating the innate immune response followed by the induction of inflammatory gene expression. Pattern-recognition receptors exemplified by toll-like receptors (TLRs) are key regulators in host response to bacterial, viral and fungal components. Central to this response is the ability to up-regulate genes involved in host protection, innate and adaptive immune cell recruitment and pathogen clearance. Although the TLR-mediated inflammatory response is critical for providing immune defense against pathogens, dysfunctional responses may lead to both acute and chronic inflammatory states manifested as sepsis, inflammation and autoimmunity. Therefore, the intensity and duration of TLR responses must be tightly controlled (Lord et al. 2009).

In addition to TLR, recent research has focused on the discovery and characterization of a new immune gene family, known as CATERPILLER or NOD-like receptor. Mutations in several NLR genes have been associated with human disease states, including autoimmunity and inflammation. Members of the NLR gene family are involved in regulating cellular activation after exposure to specific or multiple pathogen-derived products. NLR genes are involved in regulating a variety of host defense processes. The relevance of NLR genes is most apparently revealed by the genetic analysis of patients suffering from immune and inflammatory disorders (Lord et al. 2009).

Monarch-1, also called "NOD-like receptor, pyrin domain containing 12" (NLRP12), is the best known component of NLRs. NLRP12 functions as a negative regulator of TLR- and TNFR-induced NF-κB signaling in human cells. NLRP12 blocks IRAK (IL-1R-associated kinase)-1 hyperphosphorylation/ activation and facilitates the degradation of NF-κB-inducing kinase (NIK), leading to reduced NF-κB activation (Lich et al. 2007).

The result of the TLR-activated NF-κB signaling is the production of several inflammatory cytokines, including TNF-α. The overproduction of those cytokines leads to signs and symptoms of inflammatory diseases like allergies, autoimmune diseases and other acute/chronic inflammatory diseases. The most important diseases which are associated to an aberrant hyperactivation of the non canonical NF-kB pathway are summarized as follows (from Imani Fooladi et al. 2013; Delunardo et al. 2013; Meneghini et al. 2013; Lv et al. 2013; Monaco et al. 2004; Zhang et al. 2013; Medeiros, LaFerla. 2013; Qu et al. 2012; Wenhong et al. 2012; Qi et al. 2012; Mori T et al. 2012)):
- Allergic diseases, like Allergic rhinitis, Allergic conjunctivitis, Atopic allergy, Atopic dermatitis, Contact dermatitis, Eczema and/or Allergic vasculitis.
- Autoimmune diseases, like Alopecia areata, Ankylosing Spondylitis, Autoimmune cardiomyopathy, Autoimmune connective tissue diseases, Autoimmune enteropathy, Autoimmune hepatitis, Autoimmune peripheral neuropathy, Autoimmune pancreatitis, Autoimmune polyendocrine syndrome, Autoimmune thrombocytopenic purpura, Autoimmune urticaria, Autoimmune uveitis, Celiac disease, Chronic Fatigue Syndrome, Cystic fibrosis, Hashimoto's thyroiditis, Idiopathic pulmonary fibrosis, Idiopathic thrombocytopenic purpura, IgA nephropathy, Juvenile idiopathic arthritis (or Juvenile rheumatoid arthritis, or Still's disease) Kawasaki's disease, Lichen planus, Lupus erythematosus, Rheumatoid arthritis, Rheumatic fever, Sjögren's syndrome, Spondyloarthropathy, Temporal arteritis (or giant cell arteritis), Ulcerative colitis, Urticarial vasculitis, Vitiligo.
- Inflammatory diseases other than allergic or autoimmune, like Alzheimer's disease, Atherosclerosis, Chronic Liver Diseases, Chronic Nephropathy, Gastritis, Glomerulonephritis, Hepatitis A B & C, Hydradenitis suppurativa, Hypogammaglobulinemia, Interstitial cystitis, Lichen sclerosus, Liver steatosis, Metabolic Syndrome, Obesity, Parkinson's disease, Pemphigus vulgaris, Post-ischemic inflammation, Psoriasis, Psoriatic arthritis, Raynaud phenomenon, Restless leg syndrome, Retroperitoneal fibrosis, Thrombocytopenia.

Pidotimod, whose chemical name is (4R)-3-(5-oxo-L-prolyl)-1,3-thiazolidine-4-carboxylic acid, was disclosed for the first time in IT1231723. It is a synthetic peptide-like molecule provided with an in vitro and in vivo immunomodulating action (Giagulli et al., 2009). Immune system intervenes in maintaining a homeostatic balance between our body and all foreign substances; therefore, an abnormality in this function may cause a defective or aberrant response towards non-self structures, as well as loss of tolerance toward self-antigens: in any cases, the immune system error exhibits clinically as signs of disease.

Pidotimod exerts a protective action towards infectious processes (although it is free from a direct antimicrobial and antiviral action), as it has been confirmed in experimental bacterial and viral infection models and confirmed by a number of controlled clinical trials. Particularly, ex vivo studies highlighted the Pidotimod ability to increment phagocytes stimulation and relative phagocyte action, as well as the cytotoxic action of NK cells. Moreover, Pidotimod was found to increment the release of antiviral molecules (IFN-α) induced by specific stimuli (Taramelli et al. 1994).

Pidotimod, administered in tablets via oral route, proved to be able to induce a higher resistance to viral infections in animal models, as well as a higher efficiency in the treatment of recurrent respiratory infections in the pediatric patients.

### DESCRIPTION OF THE INVENTION

It has been now surprisingly found that, besides being active in sustaining protective antimicrobial immune responses Pidotimod increases transcription and synthesis of the NLRP12 (Monarch 1), involved in the control of inflammation consequent to TLR activation. This Pidotimod-induced event leads to the control of production of inflammatory mediators released by immune cells upon TLR stimulation. Therefore, through this newly discovered mechanism, Pidotimod is also able to mitigate the inflammatory pathway underlying inflammation-associated diseases characterized by an aberrant hyperactivation of the non canonical NFkB pathway, where NLRP12 plays a crucial role.

The available drug therapy acts on the late effectors of the inflammatory cascade, e.g. by binding the TNFα or by reducing its concentrations, like the monoclonal antibodies, or by suppressing the immune system like corticosteroids, which is the cause of a number of untoward effects after prolonged use.

On the contrary, by activating Monarch-1, Pidotimod is the only drug that acts at the very early stages of the inflammatory mechanism, switching off the cascade of NF-κB activation. Compared to monoclonal antibodies, its effect is much more general and not directed onto a single inflammatory cytokine. Compared to corticosteroids, pidotimod is extremely safe and does not induce the well-known toxic effects of the long term cortisone therapy.

The object of the present invention is therefore represented by the use of pidotimod, or its stereoisomers and/or a physiologically acceptable salts thereof, in the treatment of inflammation-associated diseases characterized by an aberrant hyperactivation of the non canonical NFkB pathway, such as allergic diseases, autoimmune diseases and other inflammatory diseases based on mechanisms different from allergic or autoimmune.

The allergic diseases which can be treated in accordance to the present invention may be selected from Allergic rhinitis, Allergic conjunctivitis, Atopic allergy, Atopic dermatitis, Contact dermatitis, Eczema and/or Allergic vasculitis.

The autoimmune diseases which can be treated in accordance to the present invention may be selected Alopecia areata, Ankylosing Spondylitis, Autoimmune cardiomyopathy, Autoimmune connective tissue diseases, Autoimmune enteropathy, Autoimmune hepatitis, Autoimmune peripheral neuropathy, Autoimmune pancreatitis, Autoimmune polyendocrine syndrome, Autoimmune thrombocytopenic purpura, Autoimmune urticaria, Autoimmune uveitis, Celiac disease, Chronic Fatigue Syndrome, Cystic fibrosis, Hashimoto's thyroiditis, Idiopathic pulmonary fibrosis, Idiopathic thrombocytopenic purpura, IgA nephropathy, Juvenile idiopathic arthritis (or Juvenile rheumatoid arthritis, or Still's disease) Kawasaki's disease, Lichen planus, Lupus erythematosus, Rheumatoid arthritis, Rheumatic fever, Sjögren's syndrome, Spondyloarthropathy, Temporal arteritis (or giant cell arteritis), Ulcerative colitis, Urticarial vasculitis, Vitiligo.

The inflammatory diseases based on mechanisms different from allergic or autoimmune which can be treated in accordance to the present invention may be selected from Alzheimer's disease, Atherosclerosis, Chronic Liver Diseases, Chronic Nephropathy, Gastritis, Glomerulonephritis, Hepatitis A B & C, Hydradenitis suppurativa, Hypogammaglobulinemia, Interstitial cystitis, Lichen sclerosus, Liver steatosis, Metabolic Syndrome, Obesity, Parkinson's disease, Pemphigus vulgaris, Post-ischemic inflammation, Psoriasis, Psoriatic arthritis, Raynaud phenomenon, Restless leg syndrome, Retroperitoneal fibrosis, Thrombocytopenia.

For the treatment of the present invention, pidotimod, or its stereoisomers and/or physiologically acceptable salts thereof, may be administered either systemically or topically.

When administered systemically, it may be in the form of solid or liquid formulations containing pidotimod, or its stereoisomers and/or physiologically acceptable salts thereof, together with at least a pharmaceutically acceptable excipient and/or adjuvant; such formulations may be administered by parenteral (i.e., intramuscular or intravenous) route, by enteral (i.e., oral or rectal) route, in the customary formulations suitable for administration by the different routes.

Such solid formulations to be systemically administered may have a w/w concentration in pidotimod from 50% to 90%, more preferably from 65% to 80%, most preferably from 70% to 75%.

Such liquid formulations to be systemically administered may have a w/w concentration in pidotimod from 0.5% to 20%, more preferably from 1% to 10%, most preferably from 2% to 8%.

According to an embodiment of the invention, when administered orally, the amount of pidotimod, or of its stereoisomers and/or physiologically acceptable salts thereof, may vary from 10 to 1000 mg per single dose, more preferably from 50 to 800 mg per single dose.

When administered topically, it may be in the form of solid, semisolid or liquid formulations containing pidotimod, or its stereoisomers and/or physiologically acceptable salts thereof, together with at least a pharmaceutically acceptable excipient and/or adjuvant; such formulations may be administered by dermal, ocular, auricular, nasal, buccal, intrasinal, endotracheal, vaginal, intravesical, rectal route, in the customary formulations suitable for administration by the different routes.

Such semi-solid or liquid formulations to be topically administered may have a w/w concentration in pidotimod from 0.1% to 30%, more preferably from 1% to 20%, most preferably from 5% to 15%.

Such solid formulations to be topically administered may have a w/w concentration in pidotimod from 50% to 90%, more preferably from 65% to 80%, most preferably from 70% to 75%.

The solid, semi-solid or liquid formulations to be used in accordance to the present invention may be prepared according to conventional techniques, may contain pharmaceutically acceptable excipients, adjuvants and/or carriers, and may also contain, in combination, one or more active principles with complementary or, in any case, useful activity.

The pharmaceutical compositions and the uses of the present invention will now be more fully described by the following examples. It should, however, be noted that such examples are given by way of illustration and not of limitation.

### EXAMPLE 1

Pidotimod action, which justifies the use thereof in accordance with the invention, has been shown by the experimentally testing as capable to control TLR-induced activation and inflammation on human monocytic cells an peripheral blood monocytic cells.
1. Culture of human monocytes. Peripheral blood mononuclear cells (PBMCs) were obtained by peripheral blood (freshly drawn or buffy coats). Monocytes were then derived from PBMCs by positive selection using anti-CD14 paramagnetic beads (Miltenyi Biotec) as described (Marini et al., 2008). Upon purification, cells were shortly cultured (from 24 to 72 hours) in complete culture medium (RPMI 1640 complemented with 10% of fetal bovine serum certified low endotoxin). Some experiments were performed on a human monocytic cell line (MonoMac 6), grown according to the American type culture collection suggestion.
2. Cell treatment with Pidotimod. PBMCs, purified primary human monocytes or MonoMac 6were treated with Pidotimod, at concentrations ranging from 1 to 25 µg/mL and, for different times. In some experiments, after 6 to 48 hours from the beginning of Pidotimod treatment, cells were washed and cultured for 6-24 hours in the presence and absence of TLR 1-9 optimal ligands (Human TLR1-9 Agonist kit, Invivogen).
3. Evaluation of gene expression involved in Pidotimod activity. Upon stimulation, cells were harvested and total RNA was extracted using the RNAeasy mini kit (Qiagen). RNA was then retro-transcribed and subjected to amplification. Real-time PCR was performed using the "Human "RT2 Profiler PCR microarray kit" according to the manufacturer's protocol (SABbiosciences, Qiagen). This kit allows a wide analysis of the expression of genes related to immune activation, giving a particular relevance to the expression of mediators of inflammation.
4. Immunoblot and immunoprecipitation assays. Cell lysates were prepared in lysis buffer containing a protease inhibitor cocktail (Roche). Protein lysates were solved by SDS-PAGE (8%) and transferred to nitrocellulose membranes previously blocked for 1 hour in TBS-Tween 20 (0.1%) containing 5% BSA. To detect the presence of cytoplasmic NLRP12, a first incubation with the goat polyclonal antibody to NLRP12 (Santa Cruz Biotechnology) was followed by the addiction of donkey-anti-goat-HRP secondary antibody. For immunoprecipitation studies, rabbit polyclonal antibodies to NLRP12 (Santa Cruz Biotechnology) were used to precipitate the proteins in the presence of 20µl protein A/G coated beads (protein A/G plus, Santa Cruz Biotechnology) for 12 hours at 4 °C. Protein complexes were washed four to six times, then incubated at 95 °C for 5 minutes and resolved by immunoblot as described above.
5. Transcription array. The Pidotimod ability to reduce the inflammation induced by potent danger signals as LPS or other PAMPs was highlighted by evaluating the level of mRNA specific for effector inflammatory molecules. To this scope total RNA was extracted using the semiautomated extraction system NucliSens Easymag (Biomerieux). RNA was then retro-transcribed in cDNA using the "High capacity cDNA Reverse Transcription Kit" (Applied Biosystem). To perform gene expression studies, cDNA derived from treated or untreated cells was then subjected to PCR using specific Taqman primers-probe sets (Applied Biosystem). Data obtained were analysed using the 2^{-ΔΔCt} method using Relative Quantification Study software (Applied Biosystems). The Ct values for each gene were normalized to the Ct values for β-action.
6. ELISA for the quantification of proinflammatory molecules induced by TLR stimulation. The increase of cytokine synthesis is the signal of cellular response to TLR stimulation. Pidotimod disciplines the extent of inflammation by upregulating the expression of NLRP12 which reduces the amplification of inflammatory signals triggered by TLR activation. PBMCs and/or purified monocytes are stimulated or not with an optimal concentration of each TLR ligand upon being pretreated or not with 1-25 μg/mL Pidotimod, and the supernatant of the cell cultures collected after 24 hours from the stimulation onset. The presence of IL-6, IL-8, TNF-α, MCP-1 and IL-1β was quantified in the cultural medium through ELISA assays (Endogen).

### Results

In-vitro studies showed a direct biological activity of Pidotimod on human Monomac 6 cells, and the ability thereof to control an overzealous inflammation response that is detrimental to the host. This has been carried out by *in vitro* monitoring of the inflammation steps induced by TLRs stimulation and how they are regulated by Pidotimod treatment in terms of cytokine and chemokine release. Further, monitor of pidotimod activity occurs through evaluation of NLRP12 by studying the ability of Pidotimod to increase its transcription and expression following treatment with the compound.

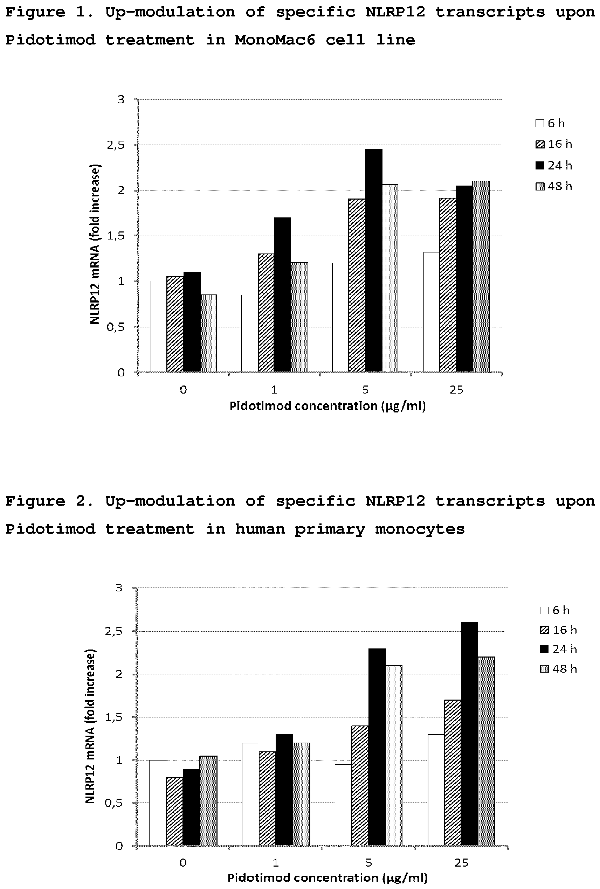

Expression of panel of genes associated to the activation of Pattern Recognition Receptor-related pathways was then analyzed by using transcriptional arrays. After 16 hours of treatment, Pidotimod (25μg/ml) was able to induce a significant increase of the NOD-like receptor NLRP12 expression. The two-fold NLRP12 mRNA increase observed in the gene array in Pidotimod-stimulated cells as compared to the unstimulated ones, was confirmed by specific quantitative Real-Time PCR assays. Analysis of cells treated with different concentrations of Pidotimod (25, 5, and 1 µg/ml) at different time points of stimulation (6, 16, 24, and 48 hours of treatment) confirmed this result, showing a peak of increase in NLRP12 expression after 24 hours of stimulation when 25 and 5 µg/ml of the molecule was used (FIGURE 1).

Primary monocytes derived from the peripheral blood of healthy donors were also susceptible to this Pidotimod effect. In all the examined samples, purified from 8 different subjects, an increase of NLRP12 mRNA expression, was observed. When compared to non-treated cells, monocytes stimulated with Pidotimod at the concentration of 25 and 5 µg/mlshowed higher levels of NLRP12 mRNA peak within 24 and 48 hours after the beginning of stimulation (FIGURE 2).

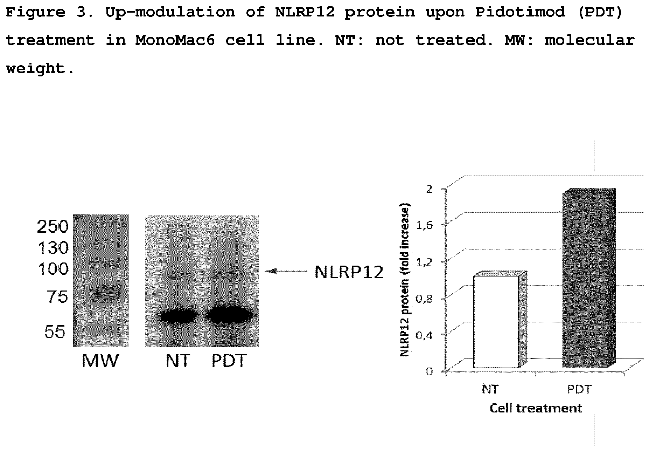

NLRP12 protein expression followed the course of mRNA expression. Monomac6 were treated for 36 hours with Pidotimod at 5 µg/ml and the NRLP12 expression level was evaluated by immunoblotting upon specific protein immunoprecipitation. A higher amount of NLRP12 protein was detected in sample derived from cells cultured in the presence of Pidotimod when compared to the untreated ones (FIGURE 3).

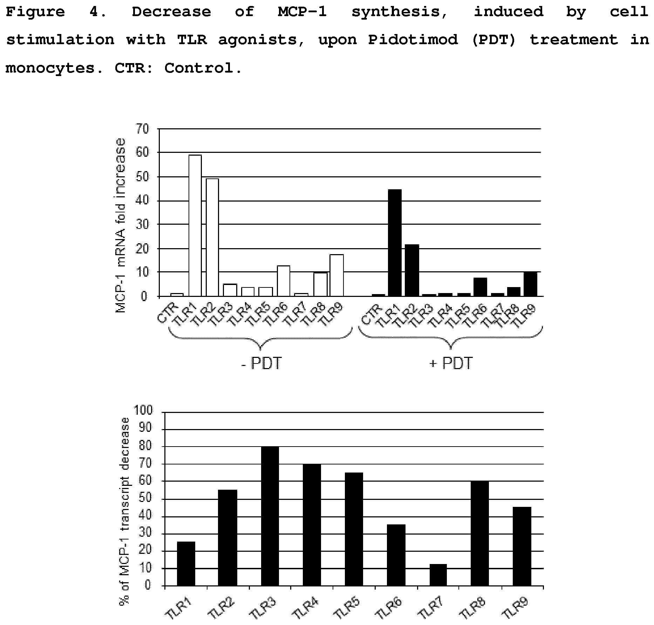

NLRP12 has been defined as a negative regulatory protein that suppresses non canonical NFkB activation and p52-dependent chemokine expression (Allen et al., 2012) and plays a critical role in dampening the potential hazardous proinflammatory activities that are activated by TLR agonists such as microbial products (Williams et al., 2005). Therefore, the effect of Pidotimod was evaluated in primary human monocytes treated with stimuli which, since they act through the TLRs, bring the monocytes to an inflammatory activated phenotype. Pidotimod treatment counteracted the extent of inflammatory mediators induced by TLR agonists. In all the samples evaluated (8 samples derived from 8 different donors), levels of specific MCP-1 consistently diminished, as representatively shown in FIGURE 4. These data were confirmed also when other key proinflammatory molecules were evaluated (IL-6, IL-8 TNF-α and IL-1β) showing that Pidotimod controls the production of these mediators at the transcriptional level

Data obtained at mRNA level were confirmed by quantification of the inflammatory molecules present in cell supernatants by ELISA. This set of results were also reproducible when total PBMC, instead of purified monocytes, were used.

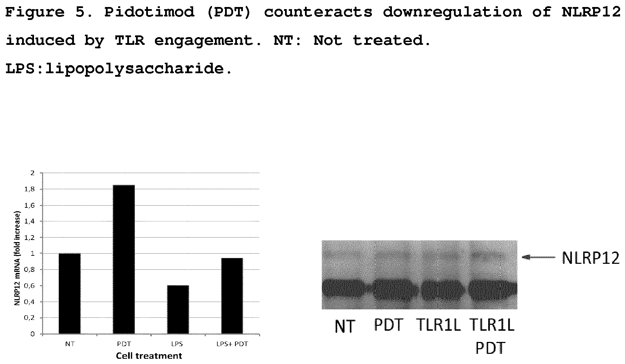

Finally, it has been assessed whether in the context of a stimulation of a representative TLR with its optimal agonist, the pretreatment of cells with Pidotimod controls the NLRP12 downregulation induced in monocytes by TLR stimulation (Williams et al., 2005). Results shown in FIGURE 5 underline how cells cultured in the presence of Pidotimod are able to counteract the decrement of NLRP12 expression both at messenger (left panel) and protein (right panel) level.

These data show that Pidotimod is able to upregulate NLRP12 expression in monocytes. Consequently, monocytes respond to NLRP12 activation by downmodulating the production of inflammatory mediators induced by TLR agonists thus controlling excessive or inappropriate inflammation that may be harmful to the host.

### EXAMPLE 2

A solution to be either injected or sprayed into the nasal cavity having the following w/V composition was prepared:

| | |
|---|---|
| 1. Pidotimod | 6.7 % |
| 2. Buffering agents | 0.05 % |
| 3. pH adjuster | q.s. to pH 6.5 |
| 4. Water for injections | q.s. to 100 % |

Preparation: the components 1 to 3 are dissolved in water for injections and mixed until a homogeneous solution is obtained with a pH of 6.5.

### EXAMPLE 3

A solution for oral administration having the following w/w % composition was prepared:

| | |
|---|---|
| 1. Pidotimod | 5.10% |
| 2. Sodium chloride | 0.07% |
| 3. Sweetener | 0.06% |
| 4. Chelating agents | 0.05% |
| 5. Tromethamine | 2.50% |
| 6. Preservatives | 0.15% |
| 7. Sweeteners solution | 31.89% |
| 8. Flavouring agents | 0.30% |
| 9. Antioxidants | 0.07% |
| 10. Colouring agents | 0.01% |
| 11. Purified water | 59.80% |

### Preparation

In a vessel dissolve the components 1 to 10 in a suitable quantity of purified water. Mix until a clear solution is obtained. Add the remaining quantity of water, mix until a homogeneous solution is obtained and filter.

### EXAMPLE 4

A tablet for oral administration having the following w/w % composition was prepared:

| | |
|---|---|
| 1. Pidotimod | 72.70% |
| 2. Diluents | 17.65% |
| 3. Disintegrants | 4.55% |
| 4. Binders | 4.00% |
| 5. Glidants | 1.10% |

### Preparation

In a vessel mix the components 1 and 2. In another vessel dissolve the component 4 in a suitable quantity of water. Mix until a clear solution is obtained. Spray the obtained solution onto mixed components 1 and 2 until a homogeneous granulate is obtained. After drying, components 3 and 5 are added to the obtained granulate and mixed until a homogeneous mixture is obtained. The mixture is then compressed by means of a tableting machine.

### EXAMPLE 5

An oil in water cream having the following w/w % composition was prepared:

| | |
|---|---|
| 1. Pidotimod | 10.00% |
| 2. Buffering agents | 5.40% |
| 3. Chelating agent | 0.10% |
| 4. Humectant | 5.00% |
| 5. Thickener | 0.25% |
| 6. Film former | 0.50% |
| 7. Emulsifiers | 15.50% |
| 8. Emollients | 13.00% |
| 9. Antioxidant | 0.50% |
| 10. Preservatives | 1.00% |
| 11. Purified Water | q.s. to 100.00% |

### Preparation

In the main vessel, solubilize components 1, 2, 3, 4, 5 in part of water. Add Xanthan Gum and disperse thoroughly until homogeneity. Separately solubilize component 7 in part of water, then add it to the main vessel while stirring. Heat the phase at 70 - 75°C. In another vessel combine the components 8, 9, 10, 11, 12 and heat at 70 - 75°C while stirring. Combine the two phases heated at the same temperature and homogenize for about 10 minutes. Cool down to 40° and add on sequence components 13 and 14, homogenizing after each addition. Cool down to room temperature under moderate stirring.

### EXAMPLE 6

A topical solution having the following w/w % composition was prepared:

| | |
|---|---|
| 1. Pidotimod | 10.00% |
| 2. Buffering agents | 5.15% |
| 3. Chelating agents | 0.10% |
| 4. Humectants | 5.00% |
| 5. Film former | 1.00% |
| 6. Purified water | q.s. to 100.00% |

### Preparation

Solubilize components 1, 2, 3, 4, 6 in water. Add component 7 and mix until clear solution is obtained.

## Claims

1. Pidotimod or its stereoisomers and/or physiologically acceptable salts thereof, for use in the treatment of inflammation-associated diseases **characterized by** an aberrant hyperactivation of the non-canonical NFkB pathway, **characterized in that** it is administered topically.

2. Pidotimod or its stereoisomers and/or physiologically acceptable salts thereof for use according to claim 1, **characterized in that** said diseases are allergic diseases, autoimmune diseases or inflammatory diseases based on mechanisms different from allergic and autoimmune.

3. Pidotimod or its stereoisomers and/or physiologically acceptable salts thereof for use according to claim 2, **characterized in that** said allergic diseases are selected from Allergic conjunctivitis, Atopic allergy, Atopic dermatitis, Contact dermatitis and/or Eczema.

4. Pidotimod or its stereoisomers and/or physiologically acceptable salts thereof for use according to claim 2, **characterized in that** said autoimmune diseases are selected from Alopecia areata, Autoimmune urticaria, Autoimmune uveitis, and/or Lichen planus.

5. Pidotimod or its stereoisomers and/or physiologically acceptable salts thereof for use according to claim 2, **characterized in that** said inflammatory diseases based on mechanisms different from allergic and autoimmune are selected from Lichen sclerosus, Pemphigus vulgaris and/or Psoriasis.

6. Pidotimod or its stereoisomers and/or physiologically acceptable salts thereof for use according to anyone of claims 1 to 5, **characterized in that** it is administered to a human.

7. Pidotimod or its stereoisomers and/or physiologically acceptable salts thereof for use according to any of the previous claims, **characterized in that** it is administered by dermal, ocular, auricular, nasal, buccal, intranasal, endotracheal, vaginal, intravescical, or rectal route.

8. Pidotimod or its stereoisomers and/or physiologically acceptable salts thereof for use according to claim 7, **characterized in that** it is administered by means of a solid, semisolid or liquid formulation.

9. Pidotimod or its stereoisomers and/or physiologically acceptable salts thereof for use according to claim 8, **characterized in that** said solid formulation has a w/w concentration in pidotimod or a physiologically acceptable salt thereof from 50% to 90%, more preferably from 65% to 80%, most preferably from 70% to 75%.

10. Pidotimod or its stereoisomers and/or physiologically acceptable salts thereof for use according to claim 8, **characterized in that** said semi-solid or liquid formulation has a w/w concentration in pidotimod or a physiologically acceptable salt thereof from 0.1 % to 30%, more preferably from 1% to 20%, most preferably from 5% to 15%.

11. Pidotimod or its stereoisomers and/or physiologically acceptable salts thereof for use according to any of the preceding claims, **characterized in that** it is administered in combination or in temporal proximity with at least one additional active principle.
